# EUROPÄISCHE PATENTANMELDUNG

(11) **EP 1 191 035 A2**
(43) Veröffentlichungstag der Anmeldung: **27.03.2002**
(21) Anmeldenummer: 01250307.4
(22) Anmeldetag: 24.08.2001
(51) Int. Cl.: C07K 14/705, C12N 15/12, C12N 15/63, C07K 16/28, G01N 33/53, C12Q 1/68, A61K 38/17, A61P 37/00, A61P 15/00, A61P 17/06

(54) **Drei Mitglieder der Zytokinrezeptorfamilie Klasse II**

(30) Priorität: 25.09.2000 DE 10048626; 17.11.2000 DE 10058907; 19.12.2000 DE 10064906
(71) Anmelder: SCHERING AKTIENGESELLSCHAFT, 13342 Berlin (DE)
(72) Erfinder: Weiss, Bertram, 14163 Berlin (DE); Sabat, Robert, 10405 Berlin (DE); Assadullah, Khusru, 14471 Postdam (DE); Toshi, Luisella, 10589 Berlin (DE)

(57) **Zusammenfassung**

Die vorliegende Erfindung betrifft drei neue Mitglieder der Zytokinrezeptor Klasse 2-Familie und ihre pharmazeutische Anwendung.

## Beschreibung

Die Erfindung betrifft drei neue Mitglieder der Zytokinrezeptorfamilie Klasse 2.

Die Zytokinrezeptorfamilie Klasse 2 wurde auf Grund der strukturellen Ähnlichkeit zwischen Proteinen definiert, die alle eine hohe Homologie zu Fibronectin Typ III aufweisen. Alle bisher beschriebenen Mitglieder dieser Familie bestehen aus drei Domänen, aus einer extrazellulären, einer transmembranären und einer intrazellulären Domäne. Die extrazelluläre Domäne umfasst ca. 200 Aminosäuren und besteht aus zwei Subdomänen mit je 100 Aminosäuren. Diese Subdomänen beinhalten konservierte Cysteine, Proline und Tryptophane, die bei der charakteristischen Faltung der Subdomänen eine Rolle spielen. Die intrazelluläre Domäne ist für die Initiierung der Signal-Transduktion verantwortlich. Der erste Schritt dafür ist die Bindung eines Liganden (z.B. Interleukin-10) an die extrazelluläre Domäne des Rezeptors 1 (z.B. IL-10R1). Es folgt die Assoziation mit dem Rezeptor 2 (z.B. IL-10R2) zu einem Komplex. Der Rezeptor 2 ist meistens nicht in der Lage alleine den Liganden zu binden, aber seine Assoziation zu dem Komplex Ligand-Rezeptor 1 ist für die Leitung des Signals ins Zellinnere/Zellkern essentiell.

Die Zytokinrezeptorfamilie Klasse 2 (CRF2) umfasst Rezeptoren für Immunmediatoren wie Interleukin 10 (IL-10), Interferon γ (IFN-γ), IFN-α und IFN-β, die für die Initiierung, Verlauf und Abschaltung von Immunreaktionen von entscheidender Bedeutung sind. Diese Mediatoren finden schon aktuell Einsatz in der Klinik.

IL-10 beispielsweise gehört zu den wichtigsten inhibitorischen Mediatoren des Immunsystems. Es hemmt die spezifische Immunantwort. Dabei scheint von besonderer Bedeutung die Beeinflussung der antigenpräsentierenden Zellen zu sein. IL-10 vermeidet die Entstehung und Reifung der Dendritischen Zellen aus Monozyten. Darüber hinaus reduziert es die Expression von MHC Klasse II und von kostimulatorischen Molekülen wie B7-2 der Monozyten und Makrophagen und somit eine adäquate Antigenpräsentation diese Zellen. Zusätzlich hemmt IL-10 die Sekretion von Interleukin-12, einem Mediator, der bei der Generierung der zellulären Immunität eine wichtige Rolle spielt. IL-10 inhibiert nicht nur die spezifische, zelluläre Immunantwort sondern beeinflusst negativ auch die unspezifische Immunreaktionen. Es hemmt die Sekretion von proinflammatorischen Zytokinen wie TNF-α, IL-1β, IL-6, IL-8, GM-CSF der Monozyten, Makrophagen und neutrophilen Granulozyten. Darüber hinaus stimuliert die Freisetzung von anti-inflammatorischen Mediatoren wie IL-1RA und lösliche TNF-α Rezeptoren. Die Bedeutung dieses Zytokines für die Limitierung der spezifischen und unspezifischen Immunreaktionen ist durch den Phänotyp der IL-10-defizienten Mäuse untermauert, die schwere Entzündungen des Darmes entwickeln, welche latent verlaufen. Diese überschiessende Immunreaktionen auf "normale" Nahrungsmittel-Antigene kann durch die IL-10 Applikation überwunden werden. Nachdem bei der ersten Applikation von IL-10 bei gesunden Probanden eine gute Verträglichkeit festgestellt wurde, findet aktuell die therapeutische Anwendung bei Erkrankungen wie z.B. Psoriasis, Morbus Crohn und Rheumatoide Arthritis statt, die durch eine starke, pathologische Immunaktivierung gekennzeichnet sind. IL-10 übt auf bestimmte Zellpopulationen wie z.B. NK-Zellen aber einen positiven Einfluss aus, was besondere Bedeutung für die Abwehr gegen virale Infektionen und Tumoren hat. Es stimuliert die zytotoxische Aktivität der NK-Zellen. Zudem steigt es die IL-2-induzierte Proliferation und Zytokinproduktion dieser Zellen.

INF-γ ist einer der potentesten Stimulatoren des Immunsystems. Es aktiviert die Monozyten und Makrophagen, verstärkt den *respiratory burst* und damit die Fähigkeit diese Zellen nicht nur phagozytierte Mikroben sondern auch unter bestimmten Bedingungen Tumorzellen abzutöten. Darüber hinaus stimuliert IFN-γ die zytotoxische Aktivität der NK-Zellen. Es verstärkt außerdem die spezifische Immunantwort. Es erhöht die Expression von MHC Klasse I und induziert bei einer ganze Reihe von Zellen die Expression von MHC Klasse II. Anschließend wirkt es direkt auf T- und B-Zellen und fördert ihre Differenzierung. Die IFN-γ-defizienten Mäuse zeigen als Ausdruck der mangelnden Aktivierung der Monozyten und Makrophagen eine hohe Empfindlichkeit gegenüber experimentellen Infektionen mit z.B. Mycobakterien, Parasieten und Viren. IFN-γ wird therapeutisch zur Rekonstruktion und zur Stimulation des Immunsystem eingesetzt.

Patientenstudien zeigten, dass ein erhöhtes Risiko für Infektionen mit Mykobakterien mit einem Defekt im Interferon-γ-Rezeptor1 (IFN-γR1) korreliert (M.J. Newport et al 1996, N Engl J Med. 335(26): 1941-9). Eine Punktmutation in diesem Rezeptor führt dazu, dass der Rezeptor nicht auf Zelloberflächen exprimiert wird. Trotzdem fanden Newport et al einen Einfluß von IFN-γ auf bestimmte Funktionen der Monozyten dieser Patienten, die den mutierten Rezeptor haben. Sie geben 3 verschiedene Hypothesen als Erklärung für diese scheinbar widersprüchlichen Ergebnisse an. Eine davon ist, dass es eventuell einen bisher nicht identifizierten zusätzlichen Rezeptor für IFN-γ gibt.

Zur gezielten Beeinflussung von Immunreaktionen ist es daher notwendig, weitere bisher unbekannte Zytokinrezeptoren zu finden.

Die vorliegende Erfindung stellt drei neue Mitglieder der Zytokinrezeptor Klasse 2-Familie bereit.

Gegenstand der vorliegenden Erfindung ist eine Nukleinsäure, welche
a. die in Seq ID No 1, Seq ID No 3 oder Seq ID No 5 dargestellten Nukleotidsequenz,
b. eine einer Sequenz aus a) im Rahmen der Degeneration des genetischen Codes entsprechende Nukleotidsequenz oder
c. eine mit den Sequenzen aus a. und/oder b. unter stringenten Bedingungen hybridisierende Nukleotidsequenz umfaßt mit der Maßgabe, dass die Nukleinsäure verschieden ist von der genomischen Sequenz und dass die Nukleinsäure für ein Polypeptid mit der biologischen Aktivität eines Zytokinrezeptors kodiert.

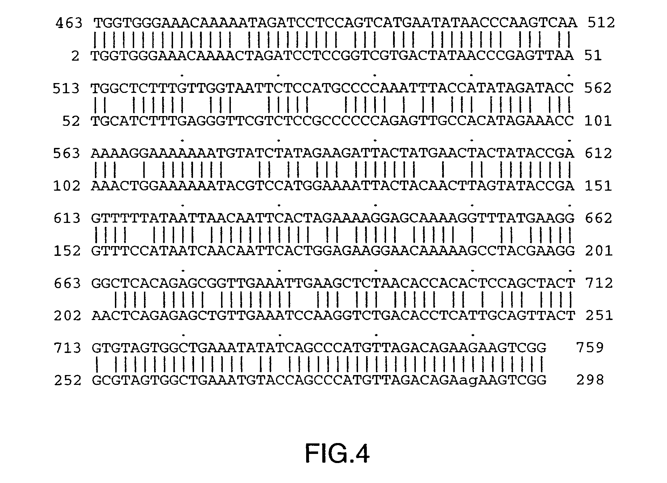

Diese Nukleinsäuren kodieren für Polypeptide, die neue Mitglieder der Familie der Zytokinrezeptoren Klasse 2 sind, oder für Abschnitte davon. Die Zytokinrezeptorfamilie Klasse 2 wird im folgenden CRF2 genannt. Diese Nukleinsäuren sind humanen Ursprungs. Die entsprechenden Sequenzen existieren auch bei anderen Säugern. Sie haben eine hohe Homologie zu den erfindungsgemäßen Sequenzen und können daher mit Hilfe der humanen Sequenz mit dem Fachmann bekannten Methoden aus entsprechenden DNA-Bibliotheken gewonnen werden. Ein Vergleich zwischen humaner und Rattensequenz ist in Abbildung 4 gezeigt.

Der Begriff "Hybridisierung unter stringenten Bedingungen" gemäß der vorliegenden Erfindung wird bei Sambrook et al. (Molecular Cloning, A Laboratory Manual, Cold Spring Harbor Laboratory Press, 1989) definiert. Ein stringente Hybridisierung liegt beispielsweise vor, wenn nach dem Waschen für 1 h mit 1 x SSC und 0,1%SDS bei 50°C, vorzugsweise bei 55°C, besonders bevorzugt bei 62°C und am meisten bevorzugt bei 68°C, insbesondere für 1h in 0,2 x SSC und 0,1 % SDS bei 55°C, vorzugsweise bei 62°C und am meisten bevorzugt bei 68°C noch ein Hybridisierungssignal beobachtet wird. Die Nukleinsäuren, die unter diesen Bedingungen mit der in Seq.ID.No1 Seq. IQNO3 oder Seq. IQN05 gezeigten Nukleinsäure oder einer dieser Sequenz im Rahmen der Degeneration des genetischen Codes entsprechende Nukleotidsequenz hybridisiert, sind auch Gegenstand der vorliegenden Erfindung.

Die erfindungsgemäße Nukleinsäure ist vorzugsweise eine DNA. Sie kann aber auch eine RNA umfassen.
Die erfindungsgemäße Nukleinsäure kann aus einer aus menschlichen Zellen hergestellten cDNA Bibliothek oder einer genomischen Bibliothek erhalten werden.

Der Begriff genomische Sequenz wird gemäß der vorliegenden Erfindung verwendet für die der erfindungsgemäßen DNA entsprechende genomische Sequenz, die sich auf Chromosom 6q24.1-25.2 befindet. In öffentlichen Datenbanken ist der Klon 503F13 (accession NO: AL050337) zugänglich. Es ist bekannt, dass dieser das Gen für den Interferon-γ-Rezeptor enthält. Es ist jedoch nicht bekannt, dass ein weiteres Gen auf diesem Chromosomenabschnitt liegt. Die vorliegende Erfindung identifiziert den Anfang (78552 bp) und das Ende des Gens (106834 bp) und drei unterschiedliche Intron/Exon-Strukturen des Gens. Die drei Intron/Exon-Strukturen führen zu drei unterschiedlichen Splice-Varianten des Gens.

Im Gegensatz zu allen bisher bekannten Mitgliedern der CRF2 sind die drei erfindungsgemäßen neuen Mitglieder dieser Familie, mit der in Seq ID No 2, Seq ID No 4 und Seq ID No 6 beschriebenen Sequenz, nicht membranständig. Sie besitzen keine transmembranäre Domäne sondern nur eine extrazelluläre Domäne. Sie werden von der Zelle, in der sie produziert werden, sezerniert und liegen dann in Körperflussigkeiten als lösliche Rezeptoren vor.

Die erfindungsgemäßen Zytokinrezeptoren können zwar ihren spezifischen Liganden, das Zytokin, binden, aber da sie lösliche Rezeptoren sind, also nicht auf einer Zellmembran verankert sind, können sie keine Signale in das Zellinnere weiterleiten. Dadurch wird die Wirkung des Zytokins eingeschränkt oder aufgehoben.
Es besteht aber auch die Möglichkeit, dass das Zytokin durch die Bindung an den löslichen Rezeptor an einen anderen Ort des Organismus transportiert wird. Es ist durch die Bindung an einen erfindungsgemäßen Zytokinrezeptor vor proteolytischem Abbau geschützt. Dadurch kann das Zytokin noch lange nach seiner Freisetzung aus Zellen und vor allem an einem anderem Ort des Oganismus seine biologische Wirkung ausüben. Dabei wird das Zytokin aus dem Komplex mit dem erfindungsgemäßen Zytokinrezeptor wieder freigelassen und kann an membranständige Rezeptoren binden.
Außerdem kann ein erfindungsgemäßer Zytokinrezeptor, nachdem er sein spezifisches Zytokin gebunden hat, mit rezeptor-negativen Zellen assoziieren und so bestimmte biologische Effekte auslösen. In diesem Fall ist die Entstehung des Komplexes aus löslichem Rezeptor und dem Zytokin die Voraussetzung für biologische Effekte des Zytokins.

Die Erfindung betrifft auch eine Nukleinsäure, welche einen Protein-kodierenden Abschnitt der in Seq ID No 1, Seq ID No 3 oder Seq ID No 5 dargestellten Nukleotidsequenz umfaßt. Die in Seq.ID No.1 dargestellte Nukleinsäure enthält einen offenen Leserahmen, der sich von Position 1 bis Position 750 erstreckt und einem Polypeptid mit der Länge von 249 Aminosäuren entspricht.

Die in Seq ID No 3 dargestellte Nukleinsäure enthält einen offenen Leserahmen, der sich von Position 304 bis Position 996 erstreckt und einem Polypeptid mit der Länge von 231 Aminosäuren enspricht.

Die in Seq ID No 5 dargestellte Nukleinsäure enthält einen offenen Leserahmen, der sich von Position 12 bis Position 800 erstreckt und einem Polypeptid mti der Länge von 263 Aminosäuren entspricht .

Gegenstand der Erfindung sind auch Nukleinsäuren, welche eine mehr als 80%ige, vorzugsweise eine mehr als 90%ige und besonders bevorzugt mehr als 95%ige Identität zu der in Seq ID No 1, Seq ID No 3 oder Seq ID No 5 dargestellten Nukleotidsequenz aufweisen.

Weiterhin betrifft die Erfindung eine erfindungsgemäße Nukleinsäure, die für einen Zytokinrezeptor Klasse 2 oder einen Abschnitt davon kodiert.

Ebenfalls Gegenstand der Erfindung sind Polypeptide, welche von einer erfindungsgemäßen Nukleinsäure kodiert werden. Die erfindungsgemäßen Polypeptide haben vorzugsweise die in SEQ ID No 2, Seq ID No 4 oder Seq ID No 6 dargestellte Aminosäuresequenz oder eine Identität von mehr als 80%, vorzugsweise von mehr als 90% und besonders bevorzugt von mehr als 95% zu der Aminosäuresequenz gemäß SEQ ID No 2, Seq ID No 4 oder Seq ID No 6.

Die Erfindung betrifft ferner einen Vektor, welcher mindestens eine Kopie einer erfindungsgemäßen Nukleinsäure oder einen Abschnitt davon aufweist. Vektoren können prokaryontische oder eukaryontische Vektoren sein. Beispiele für Vektoren sind pPRO (Clontech), pBAD (Invitrogen), pSG5 (Stratagene), pCI (Promega), pIRES (Clontech), pBAC (Clontech), pMET (Invitrogen), pBlueBac (Invitrogen). In diese Vektoren kann mit den dem Fachmann bekannten Methoden die erfindungsgemäße Nukleinsäure eingefügt werden. Die erfindungsgemäße Nukleinsäure befindet sich vorzugsweise in Verbindung mit Expressionssignalen wie z.B. Promotor und Enhancer auf dem Vektor.

Gegenstand der Erfindung ist weiterhin eine Zelle, welche mit einer erfindungsgemäßen Nukleinsäure oder einem erfindungsgemäßen Vektor transformiert ist. Als Zellen können z.B. *E. coli*, Hefe, *Pichia*, Sf9, COS, CV-1 oder BHK verwendet werden. Mit üblichen Methoden kann die Zelle mit einem Vektor, der die erfindungsgemäße Nukleinsäure enthält, transformiert werden.

Die Erfindung betrifft ebenfalls Antikörper gegen ein erfindungsgemäßes Polypeptid. Ein Antikörper gegen ein erfindungsgemäßes Polypeptid kann monoklonal oder polyklonal sein. Er kann gegen das gesamte erfindungsgemäße Polypeptid oder gegen Fragmente davon gerichtet sein. Die Gewinnung eines solchen Antikörpers erfolgt nach Standardmethoden durch Immunisierung von Versuchstieren.

Gegenstand der Erfindung ist weiterhin eine pharmazeutische Zusammensetzung, welche als aktive Komponente eine erfindungsgemäße Nukleinsäure, einen erfindungsgemäßen Vektor, eine erfindungsgemäße Zelle, eine gegen die Nukleinsäuresequenz gemäß Seq ID No 1, Seq ID No 3 oder Seq ID No 5 gerichtete antisense Nukleinsäure, ein erfindungsgemäßes Polypeptid oder einen erfindungsgemäßen Antikörper enthält.

Die erfindungsgemäße antisense Nukleinsäure ist eine DNA und/oder RNA, die komplementär zu einer erfindungsgemäßen mRNA ist. Sie kann die gesamte komlementäre Sequenz oder Teilsequenzen umfassen.

Die pharmazeutischen Zusammensetzungen der Erfindung werden mit den üblichen festen oder flüssigen Trägerstoffen oder Verdünnungsmitteln und den üblichen pharmazeutischen und technischen Hilfsstoffen entsprechend der gewünschten Applikationsart mit einer geeigneten Dosierung in an sich bekannter Weise hergestellt. Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.
Die erfindungsgemäßen pharmazeutischen Zusammensetzungen können auch in geeigneten Lösungen wie beispielsweise physiologischer Kochsalzlösung zur Anwendung kommen.
Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylabenzoat oder Benzylalkohol, zugesetzt werden.

Gegenstand der Erfindung ist ferner die Verwendung einer erfindungsgemäßen pharmazeutischen Zusammensetzung zur Diagnostik bei Immunerkrankungen oder in der Reproduktionmedizin.

Der Begriff Immunerkrankungen wird hierin verwendet für Immunerkrankungen im engeren Sinne (z.B. Autoimmunerkrankungen) und für Erkrankungen für dessen Verlauf das Immunsystem eine entscheidende Rolle spielt, wie z.B. Tumorerkrankungen und chronische/lebensbedrohende Infektionen (unzureichende Immunantwort).

Unter physiologischen Bedingungen, d.h. im gesunden Menschen, werden die erfindungsgemäßen Zytokinrezeptoren in der Plazenta und in der Brustdrüse sehr stark exprimiert. Diese ungewöhnliche gewebsspezifische Expression ermöglicht die Nutzung der erfindungsgemäßen pharmazeutischen Zusammensetzungen zur Diagnostik, Prävention und Behandlung von Reproduktions- und Immunerkrankungen.

Plazenta und Brustdrüse spielen immunologisch eine wichtige Rolle bei der Reproduktion und Entwicklung von Nachkommen. Die immunologische Balance zwischen Mutter und Kind wird fast ausschliesslich über diese zwei Organe gesteuert. Die Plazenta gewährleistet, dass der Fetus, der immunologisch eigentlich als fremd identifiziert werden müßte, nicht wie z.B. ein transplantiertes Organ abgestossen sondern toleriert wird. Dieses Phänomen der immunologischen Toleranz wird durch in der Plazenta produzierte immunmodulierende Faktoren erreicht. Gleichzeitig ist die Plazenta auch mitverantwortlich für die "normale" Entwicklung des Immunsystems des Fetus. Unmittelbar nach der Geburt muss sich der Säugling mit den immunologischen Gefahren seine Umwelt erfolgreich auseinandersetzen. Aber weil er zum diesem Zeitpunkt noch kein voll funktionstüchtiges Immunsystem besitzt, wird er von der so genannten Leihimmunität der Mutter unterstützt. Dabei spielt die Muttermilch eine entscheidende Rolle. In der Muttermilch finden sich Mediatoren, die das Immunsystem des Säuglings beeinflußen.
Die erfindungsgemäßen Zytokinrezeptoren sind Faktoren in diesen immunmodulatorischen Systemen der Plazenta und der Brustdrüse.

Unter pathophysiologischen Bedingungen wird der erfindungsgemäße Zytokinrezeptor auch in Geweben exprimiert, in denen im gesunden Zustand nur eine geringe Expression nachweisbar ist. Ein deutlicher Unterschied in der Expression ist z.B. in der Haut zu messen.

In gesunder Haut ist die Expression gering, jedoch bei der Psoriasis, einer entzündlichen Hauterkrankung mit immunologischen Hintergrund, nimmt sie deutlich zu. Die dritte Variante (Seq ID No 5) ist besonders in der Haut von Patienten mit Atopischer Dermatitis und Kutanem T-Zell-Lymphon exprimiert.

Bei der Psoriasis handelt es sich um eine wichtige Modellerkrankung, die wesentliche Gemeinsamkeiten mit anderen Immunerkrankungen, bei denen ein Typ1 Zytokinmuster von entscheidender Bedeutung ist, hat. Hierzu gehören unter anderem die Rheumatoide Arthritis, die Multiple Sklerose, entzündliche Darmerkrankungen wie Morbus Crohn und Colitis Ulcerosa, sowie die Transplantationsrejektion nach Organtransplantation. Die Psoriasis bietet die Möglichkeit, exemplarisches Wissen für diese Erkrankungen mit einem ähnlichen pathophysiologischen Mechanismus zu generieren (Asadullah et al, Drugs of Today 1999, 35(12):913-924; Romagnani, S. J Clin Immunol 1995, 15:121-9).

Diese unterschiedliche Expression in gesundem und kranken Gewebe ermöglicht die Nutzung der erfindungsgemäßen pharmazeutischen Zusammensetzung zur Diagnostik, Prävention und Behandlung von Immunerkrankungen wie Psoriasis, Rheumatoide Arthritis, Multiple Sklerose, entzündliche Darmerkrankungen wie Morbus Crohn und Colitis Ulcerosa, sowie die Transplantationsrejektion nach Organtransplantation.

Immunerkrankungen gehen häufig mit einer veränderten Expression oder einer Mutation eines Zytokinrezeptors einher. Die vorliegende Erfindung ermöglicht die Diagnostik der erfindungsgemäßen Zytokinrezeptoren bei Immunerkrankungen speziell in der Reproduktionsmedizin. So kann es z.B. durch eine fehlerhafte Immunreaktion zur Abstoßung des Fetus kommen. Die Diagnostik kann auf Basis der Nukleinsäure oder des Polypeptids erfolgen. Dazu werden Patienten entweder Blut -oder Gewebeproben entnommen. In diesen Proben kann die Menge an erfindungsgemäßen Polypeptid durch einen Immuntest bestimmt werden. Dazu werden Antikörper gegen ein erfindungsgemäßes Polypeptid oder gegen Fragmente davon hergestellt und diese dann in einem ELISA (enzyme-linked-immunosorbent assay), in einem RIA (radioimmunoassay) oder in der Immunhistochemie verwendet. Alternativ wird RNA aus den Proben gereinigt und die Menge an erfindungsgemäßer mRNA durch Northern Blot, PCR oder Chip-Hybridisierung gemessen. Bei der Chip-Hybridisierung sind auf speziellen Nukleinsäure-Chips erfindungsgemäße Nukleinsäuren oder Fragmente davon vorhanden. Die Menge an erfindungsgemäßer Nukleinsäure kann auch durch in situ Hybridisierung mit antisense-RNA, die gegen eine erfindungsgemäße Nukleinsäure gerichtet ist, bestimmt werden. Dabei kann die antisense-RNA mit digoxigenin, ³²P oder ³³P markiert sein.

Mit Hilfe der oben beschriebenen Diagnostik können auch Mutationen nachgewiesen werden. So können z.B. durch Hybridisierung der Patienten-DNA oder -RNA mit einer erfindungsgemäßen Nukleinsäuresequenz Mutationen einzelner Nukleotide nachgewiesen werden.
Die Ergebnisse der Diagnostik können dann mit dem Krankheitsbild der Patienten korreliert werden.

Die erfindungsgemäße pharmazeutischen Zusammensetzung kann zur Herstellung eines Medikaments zur Behandlung oder Prävention von Reproduktions- und Immunerkrankungen, die auf einem relativen Mangel an einem oder mehreren erfindungsgemäßen Zytokinrezeptoren beruhen, verwendet werden. Dies kann geschehen durch die Applikation von rekombinantem Protein oder den Einsatz von Gentherapie. Dabei wird ein Vektor, der eine erfindungsgemäße Nukleinsäure enthält, konstruiert und appliziert. Beispiele sind Vektoren, die aus Adenovirus, Adenovirus-associated virus, Herpes simplex virus oder SV40 abgeleitet sind. Die Gentherapie kann nach einem Protokoll wie von Gomez-Navarro et al. (Eur. J. Cancer (1999) 35, 867-885) beschrieben durchgeführt werden. Die Applikation kann lokal, d.h. direkt in das betroffene Gewebe oder systemisch, d.h. über den Blutkreislauf erfolgen. Dies führt zu einer erhöhten Expression des erfindungsgemäße Polypeptids.

Die erfindungsgemäße pharmazeutischen Zusammensetzung kann auch zur Behandlung oder Prävention von Reproduktions- und Immunerkrankungen, die auf einer relativen Überexpression eines oder mehrerer erfindungsgemäßer Zytokinrezeptoren beruhen, verwendet werden. Dazu kann eine antisense Nukleinsäure verwendet, die die Expression der erfindungsgemäßen Nukleinsäure verhindert. Weiterhin können erfindungsgemäße Antikörper verwendet werden, die die biologische Aktivität des Rezeptors hemmen.

Bevorzugt ist die Verwendung der erfindungsgemäßen pharmazeutischen Zusammensetzung zur Herstellung eines Medikaments zur Behandlung oder Prävention von Psoriasis.

Die erfindungsgemäße pharmazeutischen Zusammensetzung kann weiterhin zur Beeinflußung der Bindung eines Liganden an andere membranständige Rezeptoren verwendet werden. Ein erfindungsgemäßer Zytokinrezeptor kann z.B. einen Liganden des Rezeptors binden und dadurch die Wirkung des Liganden auf die Zelle beeinflußen. So können Immunerkrankungen, die auf eine zu hohe Konzentration des Liganden zurückzuführen sind, behandelt werden.

Ein erfindungsgemäßes Polypeptid oder eine erfindungsgemäße Nukleinsäure kann zur Auffindung von Liganden der erfindungsgemäßen Zytokinrezeptoren verwendet werden. Neben bekannten Zytokinen können auch bisher unbekannte Zytokine als Ligand den Rezeptor binden. Die Messung kann mit verschiedenen Methoden erfolgen. Eine Möglichkeit ist, das Polypeptid gemäß Seq ID No 2, Seq ID No 4 oder Seq ID No 6 oder Teile davon an eine feste Matrix zu binden, mit zu testenden Lösungen in Kontakt zu bringen und die gebundene Substanzen zu identifizieren. Zu testende Lösungen können beispielsweise Körperflüssigkeiten oder eine Mischung von synthetisch oder rekombinant hergestellten Peptiden oder Polypeptiden sein. Diese Peptide oder Polypeptide können markiert sein, z.B. mit Biotin.

Weiterhin kann ein erfindungsgemäßes Polypeptid oder eine erfindungsgemäße Nukleinsäure zur Auffindung von Modulatoren der erfindungsgemäßen Zytokinrezeptoren verwendet werden. Diese Modulatoren modulieren die Bindung des natürlichen Liganden an den Rezeptor. Sie können sowohl als Antagonisten als auch als Agonisten wirken. Antagonisten hemmen die Bindung des natürlichen Liganden an den Rezeptor und können zur Behandlung von Krankheiten eingesetzt werden, die auf eine zu starken Aktivität des Zytokins beruhen. Agonisten verstärken die Bindung des natürlichen Liganden an den Rezeptor und können bei Patienten verwendet werden, die eine verminderte Konzentration des natürlichen Liganden oder eine geringe Expression des Rezeptors aufweisen. Modulatoren können in einem Bindungsassay identifiziert werden.

Die Erfindung wird durch folgende Abbildungen und Beispiele näher erläutert.

### Abbildungen

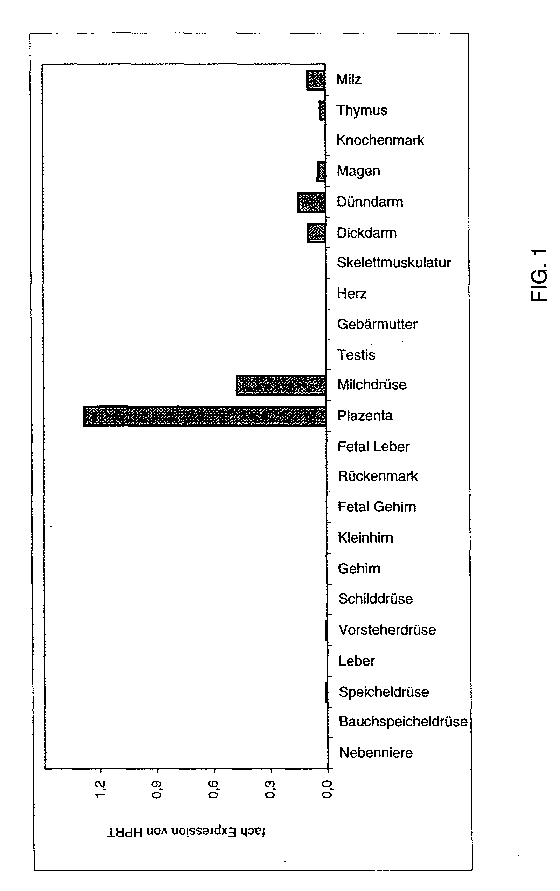

FIG 1 zeigt die Gewebsexpression der erfindungsgemäßen Zytokinrezeptoren. Aufgetragen ist die Expression als Vielfaches der Expression von HPRT

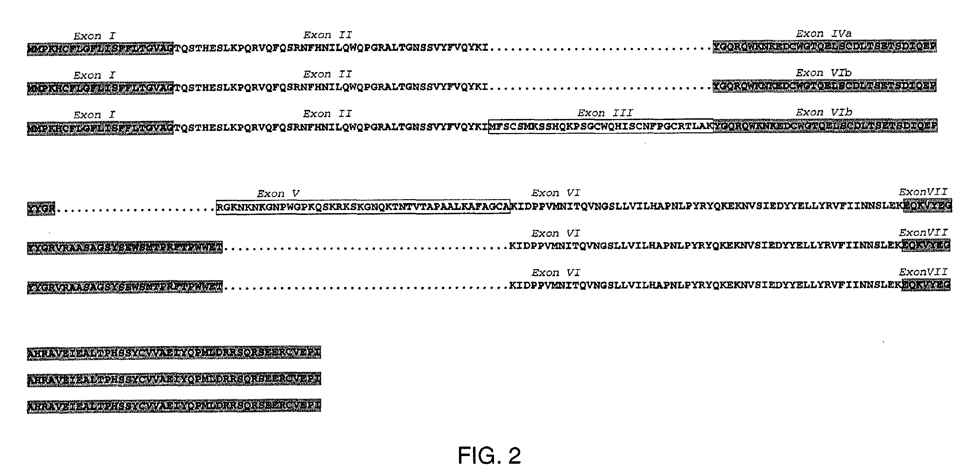

FIG 2 zeigt einen Vergleich der Aminosäuren von Seq No 2 (jeweils**1**. Zeile), Seq ID No 4 (jeweils 2. Zeile) und Seq ID No6 (jeweils 3. Zeile) und die Zuordnung der Sequenzen zu Exons.
FIG 3 zeigt die Expression des erfindungsgemäßen Zytokinrezeptors mit der in Seq ID No 5 beschriebenen Sequenz in der Haut von Patienten mit kutanem T-Zell-Lymphon (CTCL) und Atopischer Dermatitis (AD) im Vergleich zu der Haut von gesunden Kontrollprobanden.
FIG.4 zeigt einen Vergleich zwischen humanem Zytokinrezeptor (Seq ID no 3) und der entsprechenden Sequenz aus Ratte. Die jeweils obere Sequenz ist die humane Sequenz, die untere die Ratten- Sequenz.

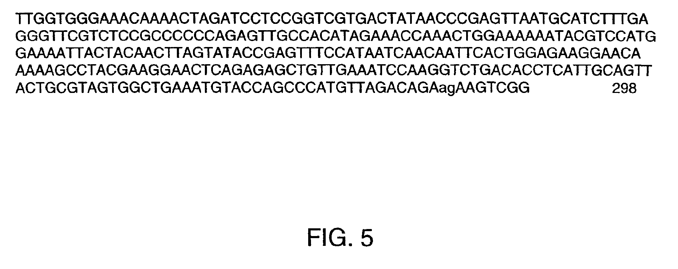

FIG.5 zeigt eine Teilsequenz des Zytokinrezeptors aus Ratte.

### Beispiele

### Beispiel 1: Nachweis eines neuen Zytokinrezeptors anhand der unterschiedlichen Wirkung von humanen IL-10 und EVB kodiertem Protein BCRF1

Es sind vier virale IL-10-Homologe beschrieben, vom Ebstein-Barr-Virus, vom Cytomegalievirus, vom equinen Herpesvirus Typ 2 und vom Ort-Virus. Das Ebstein-Barr-Virus kodiert ein Protein BCRF1, deren Aminosäurensequenz zu 83% mit humanen IL-10 (hIL-10) identisch ist. Die meisten Unterschiede beruhen auf der Deletion des N-terminalen Bereichs des humanen Zytokins und haben als Folge eine schwächere Bindung des BCRF1 am IL-10R1. BCRF1 spielt bei der Wechselwirkung zwischen dem Virus und dem infizierten Organismus, d.h. bei Ausbreitung einer EBV-Infektion eine wichtige Rolle. Wir untersuchten die biologische Wirkung von BCRF1 auf humane Monozyten, die *in vivo* die Hauptzielzellen des humanen Zytokins sind. Wir charakterisierten den Einfluss von beiden Mediatoren auf die LPS-induzierte Freisetzung von TNF-α und auf die Expression von verschiedenen Oberflächenmolekülen der Monozyten (MHC Klasse II, kostimulatorische Moleküle, Rezeptoren für Immunglobuliene). Aus dem antikoagulierten, venösen Blut der gesunden Spender wurden die Lymphozyten und die Monozyten (PBMC) durch eine Dichtengradientzentrifugation separiert. Um den inhibitorischen Einfluss von hIL-10 und BCRF1 auf die LPS-induzierte TNF-α Freisetzung zu bestimmen wurden diese Zellen 1 Stunde mit verschiedenen Konzentrationen von BCRF1 oder hlL-10 bei 37°C und in der feuchten 5% CO₂-haltigen Atmosphäre vorinkubiert. Anschließend wurden die Zellen mit 100 ng/ml LPS 3 Stunden in gleicher Umgebung stimuliert. Es folgte Abnahme des zellfreien Kulturüberstandes und die Bestimmung des TNF-α mittels ELISA. Humanes IL-10 hemmt dosisabhängig die TNF-α Freisetzung. BCRF1 übt eine ähnliche Wirkung aus, die aber wahrscheinlich auf Grund des schwächeren Bindung an IL-10R1 des viralen Homologs erst in höheren Konzentrationen (ab ca. 3 ng/ml) zum Ausdruck kommt. Wir charakterisieren auch den Einfluss der beiden Mediatoren auf die Expression von verschiedenen Oberflächenmolekülen der humanen Monozyten. Die separierten PBMC wurden 24 Stunden mit verschiedenen Konzentrationen von BCRF1 oder hIL-10 bei 37°C und in der feuchten 5% CO₂-haltigen Atmosphäre inkubiert. Anschließend wurde die Expression von den Oberflächenmolekülen der Monozyten mittels Durchflußzytometrie bestimmt. Humanes IL-10 hemmt dosisabhängig die Expression von MHC Klasse II gleichzeitig aber verstärkt die Expression der Rezeptoren für die Immunglobuline Klasse G (CD16, CD32, CD64). BCRF1 induziert wieder ähnliche Veränderungen. Interessanterweise mit hohen Konzentrationen von BCRF1 (30 ng/ml bis 100 ng/ml) induziert man eine maximale, mit hlL-10 identische Verstärkung der Expression des schwach-affinen IgG-Rezeptors CD16. Gleiche Konzentrationen von BCRF1 sind aber nicht in der Lage eine maximale Wirkung auf die Expression von dem stark-affinen IgG-Rezeptor CD64, die identisch ist mit der durch hII-10 induzierten Expression, auszuüben. Diese unterschiedliche Regulierung, in einem Fall identisch mit der von hIL-10 (CD16) im anderen Fall unterschiedlich (CD64), demonstriert die Existenz von zwei IL-10R Subtypen. Der Subtyp-1, der aus einem IL-10R1 und einem IL-10R2 besteht, ist für die Hochregulierung der Expression von CD16 verantwortlich. An ihn binden sowohl hIL-10 als auch BCRF1. Der Subtyp-2 enthält statt des IL-10R1 ein neues Mitglied der Zytokinrezeptorfamilie Klasse 2. An den Subtyp-2 bindet hIL-10R1 aber nicht BCRF1. Für die Regulierung der Expression von CD64 sind beide Subtypen verantwortlich. Weil BCRF1 den Subtyp-1 bindet, übt es auf die Expression einen gewissen Einfluss aus, aber da BCRF1 nicht in der Lage ist, den Subtyp-2 zu binden, kann es nicht die maximale, hIL-10-identische Wirkung entfalten.

### Beispiel 2: Expression in verschiedenen humanen Geweben

Die Expression der erfindungsgemäßenZytokinrezeptoren in verschiedenen humanen Geweben wurde mittels einer quantitativen RT-PCR Methode bestimmt. Dazu wurde eine "real time PCR" (TaqMan-PCR) verwendet.

Die Beseitigung von etwaiger kontaminierender genomischer DNA und die reverse Transkription der mRNA wurden wie folgt durchgeführt: 2 µg der total RNA wurden in 40 µl mit 5x moloney murine leukemia virus reverser Transkriptase (M-MLV RT) puffer (Gibco BRL, Eggenstein, Germany) und 10 mmol/L Dithiothreitol, 250 µmol/L jeweils von dATP, dUTP, dGTP und dCTP (Pharmacia biotech, Uppsala, Schweden), 1 Einheit/µL RNasin Ribonuclease Inhibitor (Promega, Mannheim, Germany), 25 ng/µL random hexadeoxynucleotide primer (Promega) und 0.05 Einheiten/µL RQ1 DNase (Promega) bei 37 °C für 30 min inkubiert. Die Proben wurden dann auf 95 °C für 10 min erhitzt und anschließend auf Eis gekühlt. Nach der Zugabe von 1 Einheit/µL RNasin Ribonuclease Inhibitor (Promega) und 5 Einheiten/µL M-MLV RT (Gibco BRL), wurden die Proben für 10 min bei Raumtemperatur und folgend für eine 1 h bei 37 °C inkubiert. Anschließend wurden die Enzyme durch Erhitzen auf 95 °C für 10 min inaktiviert. Die resultierende cDNA wurde in jeweils 3 Ansätzen (3fach Bestimmung) mittels der real-time TaqMan PCR unter Verwendung eines ABI Prism 7700 Sequence Detector System (Perkin-Elmer Cetus, Forster City, C.A. , U.S.A.) analysiert. Für die Detektion der cDNA Sequenz aller drei Varianten des erfindungsgemäßen Zytokinrezeptors wurden die folgenden PCR primer und eine fluorochrom-markierte Oligonucleotidsonde, unter Verwendung der Primer Express software (Perkin-Elmer Cetus) konstruiert: 
sense primer: CTCAGTCAACGCATGAGTCTCTG
antisense primer: CAGGCTGCCATTGCAAAAT
Sonde: FAM-AGCCTCAGAGGGTACAATTTCAGTCCCG-TAMRA.
 FAM steht für 6-Carboxy-fluorescein, TAMRA für 6-Carboxy-tetramethyl-rhodamine

Zudem wurde der mRNA Gehalt der kodierend für das "house keeping gene" *Homo sapiens* hypoxanthine phosphoribosyltransferase-1 (HPRT-1) ist und als interne Kontrolle gilt parallel in jeder einzelnen Probe bestimmt. Die Sequenz des HPRT-1-spezifischen primer Paares und die fluorogene Sonde, basierend auf den reversen komplimentären cDNA strang, war wie folgt: 
AGTCTGGCTTATATCCAACACTTCG (sense primer),
GACTTTGCTTTCCTTGGTCAGG (antisense primer),
FAM-TTTCACCAGCAAGCTTCGACCTTGA-TAMRA (sense Sonde).
 Die PCR Amplifikation wurde unter Verwendung von 1 µL reversen transcriptions Mix in einem Endvolumen von 50 µL, der den TaqMan universal master mix (Applied Biosystems, Weiterstadt, Germany) und 200 nmol/L der spezifischen Sonde und 900 (CRF2-n3) bzw. 300 (HPRT-1) nmol/L des jeweiligen Primers enthielt, durchgeführt. Die genannten Konzentrationen waren zuvor in Vorversuchen als optimal identifiziert worden. Signifikante Fluorescence Signale wurden definiert als der Schwellenwert Zyklus der mittels der TaqMan instrument-assoziierten Software version 1.6.3. (Applied Biosystems) berechnet wurde.Es wurde der Zytokinrezeptor-mRNA Gehalt berechnet in Relation zu dem für HPRT-1.
Die Gewebsspezifische Analyse der Genexpression zeigte, dass die erfindungsgemäßen Zytokinrezeptoren vornehmlich in der Plazenta und in der Brustdrüse (hier sogar stärker als das House keeping Gene) exprimiert werden .

### Beispiel 3: Expression unter pathophysiologischen Bediengungen

Die Untersuchung sollte die Fragen beantworten, ob die Expression der erfindungsgemäßen Zytokinrezeptoren regulierbar ist und ob sie sich zwischen physiologischen und pathophysiologischen Zuständen unterscheidet. Dazu wurde RNA aus Hautbiopsien von gesunden Kontrollprobanden mit RNA aus Hautbiopsien von Patienten mit Psoriasis, Atopischer Dermatitis (AD) und kutanes T-Zell-Lymphon (CTCL) verwendet. Die Bestimmung wurde wie im Beispiel 2 beschrieben durchgeführt.
Die Bestimmung ergab eine über 10-fach stärkere Expression der erfindungsgemäßen Zytokinrezeptoren in läsionaler Haut von Patienten mit Psoriasis in Vergleich zu Haut der gesunden Kontrollprobanden.
In einem weiteren Versuch wurden Sonden verwendet, die zwischen der zweiten (Seq ID No 3) und dritten Variante (Seq ID No 5) unterscheiden können:
Variante 2 (Seq ID No3): 
sense primer: CTCAGTCAACGCATGAGTCTCTG
antisense primer: CAGGCTGCCATTGCAAAAT
sonde: FAM-5'-TGCAGTACAAAATATATGGACAGAGACAATGGAAAAA-3'-TAMRA.
 Variante 3 (Seq ID No 5):

sense primer: 5'-AGGCTGCAGAACATTGGCTAA-3'
antisense primer: 5'-TTCACTGGTAAGGTCACAAGAGAGTT-3'
sonde: FAM-5'-TGGACAGAGACAATGGAAAAATAAAGAAGACTGTTG-3'-TAMRA

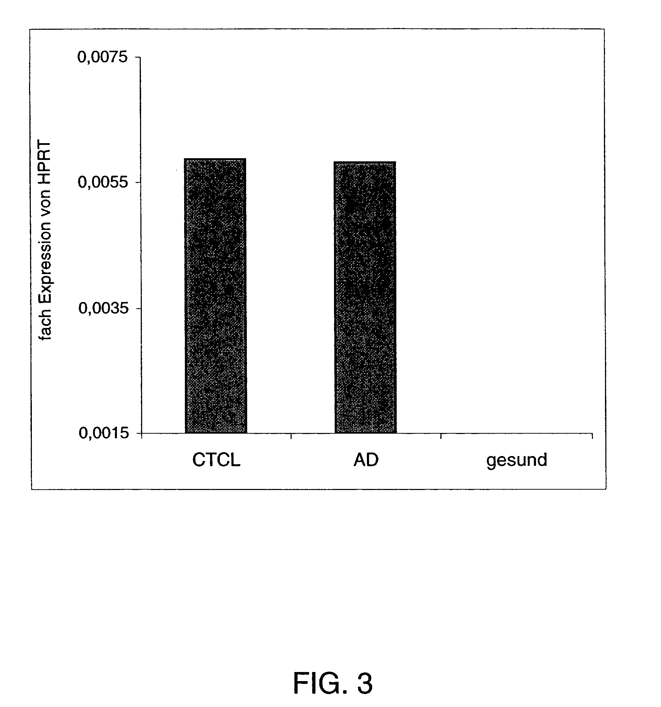

Die Expression der dritten Variante (Seq ID No5) ist im Vergleich zu gesunder Haut bei CTCL und AD deutlich erhöht (s. FIG 3).

### Beispiel 4: Klonierung der Zytokinrezeptoren gemäß Seq ID No1 und No 3

Zunächst wurde eine cDNA Bibliothek aus humaner Plazenta hergestellt. Dazu wurden 4 µg total RNA (Firma Clontech) in den Reaktionen des GeneRacer™ kit (Invitrogen, CA USA) verwendet. Die modifizierte mRNA aus der letzten Reaktion wurde mit dem GeneRacer™ OligodT primer für die reverse Transkription eingesetzt.

Die cDNA für den Zytokinrezeptor wurde mittels einer RACE-PCR (RACE- Rapid Amplification of cDNA Ends) gewonnen. Die DNA-Polymerase für die PCR war das ThermoZyme™ von Invitrogen.
5'- und 3'-RACE wurden wie folgt durchgeführt:

| 5'-RACE Ansatzvolumen | 50 µl |
|---|---|
| Plazenta cDNA | 2 µl |
| GeneRacer™ 5' primer (10 µM) | 1 µl |
| Genspezifischer primer (GSP) #2R (25 µM)1 | µl |
| 5x ThermoZyme™ PCR Puffer | 10 µl |
| dNTP Lösung(10 mM je) | 1 µl |
| ThermoZyme™ (1 U/µl) | 1 µl |
| Steriles Wasser | 34 µl |

| 3'-RACE Ansatzvolumen | 50 µl |
|---|---|
| Plazenta cDNA | 2 µl |
| GeneRacer™ 3' primer (10 µM) | 1 µl |
| GSP #1F (35 µM) | 1 µl |
| 5x ThermoZyme™ PCR Puffer | 10 µl |
| dNTP Lösung(10mM je) | 1 µl |
| ThermoZyme™ ( 1 U/µl) | 1 µl |
| Steriles Wasser | 34 µl |

Die folgende "cycling" Programme für die RACE-PCR wurden in den Thermocycler Perkin-Elmer 9600 eingestellt:

| | | |
|---|---|---|
| 94° C | 2 min | 1 cycle |
| 94° C | 30 sec | 5 cycles |
| 72° C | 3 min | |
| 94° C | 30 sec | 5 cycles |
| 70° C | 30 sec | |
| 72° C | 3 min | |

| | | |
|---|---|---|
| 94° C | 30 sec | 25 cycles |
| 68° C | 30 sec | |
| 72° C | 3 min | |
| 72° C | 10 min | 1 cycle |

Die Sequenzen der Zytokinrezeptor-primer für die RACE Reaktionen waren wie folgt:

#1F
5'- CTC AGT CAA CGC ATG AGT CTC TGA AGC –3' (sense primer)

#2R
5'- ATA GAC ACT GCT GTT GCC AGT AAG TGC C –3' (antisense primer)

Anschließend wurden die Produkte der RACE in dem pCR®4-TOPO Vektor aus dem TOPO-TA cloning® kit von Invitrogen kloniert.
Die Sequenz der klonierten PCR-Produkte wurde mittels des Applied Biosystem (ABI) Prism® BigDye™ Terminator sequencing kit in einem ABI 310 capillary Sequencer bestimmt.

### Beispiel 6: Klonierung des Zytokinrezeptors gemäß Seq ID No 5

Der Zytokinrezeptor wurde aus humaner Plazenta und Brustdrüse cDNA Bibliothek identifiziert. Die Plazenta cDNA Bibliothek wurde mittels des GeneRacer™ kit (Invitrogen, CA - USA) hergestellt. Die Brustdrüse cDNA Bibliothek war von der Firma Clontech (Marathon-Ready™cDNA).
Die cDNA für den Zytokinrezeptor wurde mittels eine PCR Amplifikation (RT-PCR) gewonnen.
Die DNA-Polymerase für die PCR war das Advantage® 2 von CLONTECH.
Die RT-PCR wurde wie folgt durchgeführt:

| RT-PCR Ansatzvolumen | 50 µl |
|---|---|
| Plazenta/Brustdrüse cDNA | 2 µl |
| CRF2-n4 #1a-F primer (20 µM) | 1 µl |
| CRF2-n4 #2b-R primer (20 µM) | 1 µl |
| 10x cDNA PCR reaction buffer | 5 µl |
| dNTP mix (10 mM) | 1 µl |
| 50x Advantage® 2 Polymerase Mix | 1 µl |
| Steriles Wasser | 36 µl |

Die folgende "cycling" Programme für die RT-PCR wurden in den Thermocycler Perkin-Elmer 9600 eingestellt:

| | | |
|---|---|---|
| 94° C | 30 sec | 1 cycle |
| 94° C | 5 sec | 30 cycles |
| 68° C | 3 min | |

Die Sequenzen der CRF2-n4 primer für die RT-PCR waren wie folgt:

#1a-F

5'- CAC TTG CAA CCA TGA TGC CTA AAC A – 3'   (sense primer)

#2b-R

5'- CCA CAA GTC ATG GAA TTT CCA CAC A – 3'   (antisense primer)

Anschließend wurden die Produkte der RT-PCR in dem pCR®4-TOPO Vektor aus dem TOPO-TA cloning® kit von Invitrogen kloniert.
Die Sequenz der klonierten PCR-Produkte wurde mittels des Applied Biosystem (ABI) Prism® BigDye™ Terminator sequencing kit in einem ABI 310 capillary Sequencer bestimmt.

### Beispiel 6: Expression der Zytokinrezeptoren

Um die gereinigten rekombinanten Proteine zu gewinnen, wurden die entsprechenden cDNAs in einem E.coli Expressionsvektor kloniert.
Die Zytokinrezeptoren wurden als Fusionsproteine mit der Glutathione-S-transferase (GST) in dem Vektor pGEX-2T (Pharmacia Biotech) exprimiert. Das Fusionskonstrukt wurde anschließend in den E.coli Stamm BL21 transformiert.

### Beispiel 7: Suche nach Modulatoren

Die erfindungsgemäßen Zytokinrezeptoren werden in E.coli exprimiert und mit Hilfe von Standardmethoden wie z.B. lonenaustauschchromatographie, Affinitätschromatographie oder Gelfiltration gereinigt. Das gereinigte Protein wird auf Mikrotiterplatten gebunden. Dann wird gleichzeitig der Biotin-markierte Ligand und die zu testenden Substanz zugegeben und 1 Stunde inkubiert. Anschließend wird die Mikrotiterplatte mit Puffer gewaschen und dann Avidin-FITC zugegeben. Nach weiteren 30 min Inkubation wird wieder gewaschen und dann die Fluoreszenz gemessen. In Kontrollansätzen wird nur der markierte Ligand zugegeben. Durch Messung der Fluoreszenz kann bestimmt werden, ob die zu testende Substanz den Liganden vom Rezeptor verdrängt (Fluoreszenz wird geringer), keinen Einfluß hat (Fluoreszenz entspricht der des Kontrollansatzes) oder die Bindung des Liganden verstärkt (Fluoreszenz wird stärker). Analoge Versuche werden mit ¹²⁵J markiertem Liganden durchgeführt.

## Patentansprüche

1. Nukleinsäure, **dadurch gekennzeichnet, dass** sie
a. die in Seq ID No 1, Seq ID No 3 oder Seq ID No 5 dargestellte Nukleotidsequenz,
b. eine einer Sequenz aus a) im Rahmen der Degeneration des genetischen Codes entsprechende Nukleotidsequenz oder
c. eine mit den Sequenzen aus a. und/oder b. unter stringenten Bedingungen hybridisierende Nukleotidsequenz, die für ein Polypeptid mit der biologischen Aktivität eines Zytokinrezeptors kodiert
umfaßt mit der Maßgabe, dass die Nukleinsäure verschieden ist von der genomischen Sequenz.

2. Nukleinsäure nach Anspruch 1, **dadurch gekennzeichnet, dass** sie einen Protein-kodierenden Abschnitt der in Seq ID No 1, Seq ID No 3 oder Seq ID No 5 dargestellten Nukleotidsequenz umfaßt.

3. Nukleinsäure nach Anspruch 1 oder 2, **dadurch gekennzeichnet, dass** sie eine mehr als 80%ige Identität zu der in Seq ID No 1, Seq ID No 3 oder Seq ID No 5 dargestellten Nukleotidsequenz aufweist.

4. Nukleinsäure nach einem der Ansprüche 1-3, **dadurch gekennzeichnet, dass** sie für einen Zytokinrezeptor Klasse 2 oder einen Abschnitt davon kodiert.

5. Polypeptid kodiert von einer Nukleinsäure nach einem der Ansprüche 1-4.

6. Polypeptid nach Anspruch 5 **dadurch gekennzeichnet, dass** es die in SEQ ID No 2, Seq ID No 4 oder Seq ID No 6 dargestellte Aminosäuresequenz aufweist.

7. Vektor, **dadurch gekennzeichnet, dass** er mindestens eine Kopie einer Nukleinsäure nach einem der Ansprüche 1-4 oder einen Abschnitt davon aufweist.

8. Zelle, **dadurch gekennzeichnet, dass** sie mit einer Nukleinsäure nach einem der Ansprüche 1-4 oder einem Vektor nach Anspruch 8 transformiert ist.

9. Antikörper gegen ein Polypeptid nach einem der Ansprüche 5-7

10. Pharmazeutische Zusammensetzung, **dadurch gekennzeichnet, dass** sie als aktive Komponente
a. eine Nukleinsäure nach einem der Ansprüche 1-4,
b. einen Vektor nach Anspruch 7,
c. eine Zelle nach Anspruch 8,
d. eine gegen die Nukleinsäuresequenz gemäß Seq ID No 1, Seq ID No 3 oder Seq ID No 5 gerichtete antisense Nukleinsäure,
e. ein Polypeptid nach einem der Ansprüche 5-6
oder
f. einen Antikörper gemäß Anspruch 9 enthält.

11. Verwendung einer pharmazeutischen Zusammensetzung nach Anspruch 10 zur Diagnostik bei Immunerkrankungen oder in der Reproduktionmedizin.

12. Verwendung einer pharmazeutischen Zusammensetzung nach Anspruch 11 zur Herstellung eines Medikaments zur Behandlung und Prävention von Reproduktions- oder Immunkrankheiten.

13. Verwendung nach Anspruch 12 wobei die Immunkrankheit Psoriasis ist.

14. Verwendung eines Polypeptids nach einem der Ansprüche 5-6 oder einer Nukleinsäure nach einem der Ansprüche 1-4 zur Auffindung von Liganden eines Polypeptids nach Anspruch 5.

15. Verwendung eines Polypeptids nach einem der Ansprüche 5-6 oder einer Nukleinsäure nach einem der Ansprüche 1-4 zur Auffindung von Modulatoren eines Polypeptids nach Anspruch 5.


SEQUENZPROTOKOLL

<110> Schering AG

<120> Drei neue Mitglieder der Zytokinrezeptorfamilie Klasse
      2

<130> 52023EP

<140>
<141>

<160> 6

<170> PatentIn Ver. 2.1

<210> 1
<211> 750
<212> DNA
<213> Homo sapiens

<400> 1
atgatgccta aacattgctt tctaggcttc ctcatcagtt tcttccttac tggtgtagca 60
ggaactcagt caacgcatga gtctctgaag cctcagaggg tacaatttca gtcccgaaat 120
tttcacaaca ttttgcaatg gcagcctggg agggcactta ctggcaacag cagtgtctat 180
tttgtgcagt acaaaatata tggacagaga caatggaaaa ataaagaaga ctgttggggt 240
actcaagaac tctcttgtga ccttaccagt gaaacctcag acatacagga accttattac 300
gggaggaggg gcaaaaataa aaataaaggg aatccttggg ggccaaaaca aagtaaacgg 360
aaatcaaagg ggaaccagaa gaccaacaca gtgactgccc cagctgccct gaaggcattt 420
gctggatgtg caaaaataga tcctccagtc atgaatataa cccaagtcaa tggctctttg 480
ttggtaattc tccatgctcc aaatttacca tatagatacc aaaaggaaaa aaatgtatct 540
atagaagatt actatgaact actataccga gtttttataa ttaacaattc actagaaaag 600
gagcaaaagg tttatgaagg ggctcacaga gcggttgaaa ttgaagctct aacaccacac 660
tccagctact gtgtagtggc tgaaatatat cagcccatgt tagacagaag aagtcagaga 720
agtgaagaga gatgtgtgga aattccatga                                  750

<210> 2
<211> 249
<212> PRT
<213> Homo sapiens

<400> 2
Met Met Pro Lys His Cys Phe Leu Gly Phe Leu Ile Ser Phe Phe Leu
  1               5                  10                  15

Thr Gly Val Ala Gly Thr Gln Ser Thr His Glu Ser Leu Lys Pro Gln
             20                  25                  30

Arg Val Gln Phe Gln Ser Arg Asn Phe His Asn Ile Leu Gln Trp Gln
         35                  40                  45

Pro Gly Arg Ala Leu Thr Gly Asn Ser Ser Val Tyr Phe Val Gln Tyr
     50                  55                  60

Lys Ile Tyr Gly Gln Arg Gln Trp Lys Asn Lys Glu Asp Cys Trp Gly
 65                  70                  75                  80

Thr Gln Glu Leu Ser Cys Asp Leu Thr Ser Glu Thr Ser Asp Ile Gln
                 85                  90                  95

Glu Pro Tyr Tyr Gly Arg Arg Gly Lys Asn Lys Asn Lys Gly Asn Pro
            100                 105                 110

Trp Gly Pro Lys Gln Ser Lys Arg Lys Ser Lys Gly Asn Gln Lys Thr
        115                 120                 125

Asn Thr Val Thr Ala Pro Ala Ala Leu Lys Ala Phe Ala Gly Cys Ala
    130                 135                 140

Lys Ile Asp Pro Pro Val Met Asn Ile Thr Gln Val Asn Gly Ser Leu
145                 150                 155                 160

Leu Val Ile Leu His Ala Pro Asn Leu Pro Tyr Arg Tyr Gln Lys Glu
                165                 170                 175

Lys Asn Val Ser Ile Glu Asp Tyr Tyr Glu Leu Leu Tyr Arg Val Phe
            180                 185                 190

Ile Ile Asn Asn Ser Leu Glu Lys Glu Gln Lys Val Tyr Glu Gly Ala
        195                 200                 205

His Arg Ala Val Glu Ile Glu Ala Leu Thr Pro His Ser Ser Tyr Cys
    210                 215                 220

Val Val Ala Glu Ile Tyr Gln Pro Met Leu Asp Arg Arg Ser Gln Arg
225                 230                 235                 240

Ser Glu Glu Arg Cys Val Glu Ile Pro
                245

<210> 3
<211> 1255
<212> DNA
<213> Homo sapiens

<400> 3
aactcacttt accactactc gctatagagc cctggtcaag ttctctccac ctctctatct 60
atgtctcagt ttcttcatct gtaacatcaa atgaataata ataccaatct cctagacttc 120
ataagaggat taacaaagac aaaatatggg aaaaacataa catggcgtcc cataattatt 180
agatcttatt attgacacta aaatggcatt aaaattacca aaaggaagac agcatctgtt 240
tcctctttgg tcctgagctg gttaaaagga acactggttg cctgaacagt cacacttgca 300
accatgatgc ctaaacattg ctttctaggc ttcctcatca gtttcttcct tactggtgta 360
gcaggaactc agtcaacgca tgagtctctg aagcctcaga gggtacaatt tcagtcccga 420
aattttcaca acattttgca atggcagcct gggagggcac ttactggcaa cagcagtgtc 480
tattttgtgc agtacaaaat atatggacag agacaatgga aaaataaaga agactgttgg 540
ggtactcaag aactctcttg tgaccttacc agtgaaacct cagacataca ggaaccttat 600
tacgggaggg tgagggcggc ctcggctggg agctactcag aatggagcat gacgccgcgg 660
ttcactccct ggtgggaaac aaaaatagat cctccagtca tgaatataac ccaagtcaat 720
ggctctttgt tggtaattct ccatgctcca aatttaccat atagatacca aaaggaaaaa 780
aatgtatcta tagaagatta ctatgaacta ctataccgag tttttataat taacaattca 840
ctagaaaagg agcaaaaggt ttatgaaggg gctcacagag cggttgaaat tgaagctcta 900
acaccacact ccagctactg tgtagtggct gaaatatatc agcccatgtt agacagaaga 960
agtcagagaa gtgaagagag atgtgtggaa attccatgac ttgtggaatt tggcattcag 1020
caatgtggaa attctaaagc tccctgagaa caggatgact cgtgtttgaa ggatcttatt 1080
taaaattgtt tttgtatttt cttaaagcaa tattcactgt tacaccttgg ggacttcttt 1140
gtttacccat tcttttatcc tttatatttc atttgtaaac tatatttgaa cgacattccc 1200
cccgaaaaat tgaaatgtaa agatgaggca gagaataaag tgttctatga aatgc 1255

<210> 4
<211> 231
<212> PRT
<213> Homo sapiens

<400> 4
Met Met Pro Lys His Cys Phe Leu Gly Phe Leu Ile Ser Phe Phe Leu
1               5                   10                  15

Thr Gly Val Ala Gly Thr Gln Ser Thr His Glu Ser Leu Lys Pro Gln
            20                  25                  30

Arg Val Gln Phe Gln Ser Arg Asn Phe His Asn Ile Leu Gln Trp Gln
        35                  40                  45

Pro Gly Arg Ala Leu Thr Gly Asn Ser Ser Val Tyr Phe Val Gln Tyr
    50                  55                  60

Lys Ile Tyr Gly Gln Arg Gln Trp Lys Asn Lys Glu Asp Cys Trp Gly
65                  70                  75                  80

Thr Gln Glu Leu Ser Cys Asp Leu Thr Ser Glu Thr Ser Asp Ile Gln
                85                  90                  95

Glu Pro Tyr Tyr Gly Arg Val Arg Ala Ala Ser Ala Gly Ser Tyr Ser
            100                 105                 110

Glu Trp Ser Met Thr Pro Arg Phe Thr Pro Trp Trp Glu Thr Lys Ile
        115                 120                 125

Asp Pro Pro Val Met Asn Ile Thr Gln Val Asn Gly Ser Leu Leu Val
    130                 135                 140

Ile Leu His Ala Pro Asn Leu Pro Tyr Arg Tyr Gln Lys Glu Lys Asn
145                 150                 155                 160

Val Ser Ile Glu Asp Tyr Tyr Glu Leu Leu Tyr Arg Val Phe Ile Ile
                165                 170                 175

Asn Asn Ser Leu Glu Lys Glu Gln Lys Val Tyr Glu Gly Ala His Arg
            180                 185                 190

Ala Val Glu Ile Glu Ala Leu Thr Pro His Ser Ser Tyr Cys Val Val
        195                 200                 205

Ala Glu Ile Tyr Gln Pro Met Leu Asp Arg Arg Ser Gln Arg Ser Glu
    210                 215                 220

Glu Arg Cys Val Glu Ile Pro
225                 230


<210> 5
<211> 810
<212> DNA
<213> Homo sapiens

<400> 5
cacttgcaac catgatgcct aaacattgct ttctaggctt cctcatcagt ttcttcctta 60
ctggtgtagc aggaactcag tcaacgcatg agtctctgaa gcctcagagg gtacaatttc 120
agtcccgaaa ttttcacaac attttgcaat ggcagccggg gagggcactt actggcaaca 180
gcagtgtcta ttttgtgcag tacaaaatca tgttctcatg cagcatgaaa agctctcacc 240
agaagccaag tggatgctgg cagcacattt cttgtaactt cccaggctgc agaacattgg 300
ctaaatatgg acagagacaa tggaaaaata aagaagactg ttggggtact caagaactct 360
cttgtgacct taccagtgaa acctcagaca tacaggaacc ttattacggg agggtgaggg 420
cggcctcggc tgggagctac tcagaatgga gcatgacgcc gcggttcact ccctggtggg 480
aaacaaaaat agatcctcca gtcatgaata taacccaagt caatggctct ttgttggtaa 540
ttctccatgc cccaaattta ccatatagat accaaaagga aaaaaatgta tctatagaag 600

attactatga actactatac cgagttttta taattaacaa ttcactagaa aaggagcaaa 660
aggtttatga aggggctcac agagcggttg aaattgaagc tctaacacca cactccagct 720
actgtgtagt ggctgaaata tatcagccca tgttagacag aagaagtcgg agaagtgaag 780
agagatgtgt ggaaattcca tgacttgtgg 810

<210> 6
<211> 263
<212> PRT
<213> Homo sapiens

<400> 6
Met Met Pro Lys His Cys Phe Leu Gly Phe Leu Ile Ser Phe Phe Leu
1               5                   10                  15

Thr Gly Val Ala Gly Thr Gln Ser Thr His Glu Ser Leu Lys Pro Gln
            20                  25                  30

Arg Val Gln Phe Gln Ser Arg Asn Phe His Asn Ile Leu Gln Trp Gln
        35                  40                  45

Pro Gly Arg Ala Leu Thr Gly Asn Ser Ser Val Tyr Phe Val Gln Tyr
    50                  55                  60

Lys Ile Met Phe Ser Cys Ser Met Lys Ser Ser His Gln Lys Pro Ser
65                  70                  75                  80

Gly Cys Trp Gln His Ile Ser Cys Asn Phe Pro Gly Cys Arg Thr Leu
                85                  90                  95

Ala Lys Tyr Gly Gln Arg Gln Trp Lys Asn Lys Glu Asp Cys Trp Gly
            100                 105                 110

Thr Gln Glu Leu Ser Cys Asp Leu Thr Ser Glu Thr Ser Asp Ile Gln
        115                 120                 125

Glu Pro Tyr Tyr Gly Arg Val Arg Ala Ala Ser Ala Gly Ser Tyr Ser
    130                 135                 140

Glu Trp Ser Met Thr Pro Arg Phe Thr Pro Trp Trp Glu Thr Lys Ile
145                 150                 155                 160

Asp Pro Pro Val Met Asn Ile Thr Gln Val Asn Gly Ser Leu Leu Val
                165                 170                 175

Ile Leu His Ala Pro Asn Leu Pro Tyr Arg Tyr Gln Lys Glu Lys Asn
            180                 185                 190

Val Ser Ile Glu Asp Tyr Tyr Glu Leu Leu Tyr Arg Val Phe Ile Ile
        195                 200                 205

Asn Asn Ser Leu Glu Lys Glu Gln Lys Val Tyr Glu Gly Ala His Arg
    210                 215                 220

Ala Val Glu Ile Glu Ala Leu Thr Pro His Ser Ser Tyr Cys Val Val
225                 230                 235                 240

Ala Glu Ile Tyr Gln Pro Met Leu Asp Arg Arg Ser Arg Arg Ser Glu
                245                 250                 255

Glu Arg Cys Val Glu Ile Pro
            260